# EUROPEAN PATENT APPLICATION

(11) **EP 4 372 010 A1**
(43) Date of publication of application: **22.05.2024**
(21) Application number: 22841336.5
(22) Date of filing: 12.07.2022
(51) Int. Cl.: C07K 19/00, C12N 5/10, A61K 39/00, A61P 35/00

(54) **NOVEL CHIMERIC RECEPTOR COMPOSITION, RECOMBINANT VECTOR, CELL, AND APPLICATION THEREOF**

(30) Priority: 14.07.2021 CN 202110793058
(71) Applicant: Suzhou Immunofoco Biotechnology Co., Ltd., Suzhou, Jiangsu 201210 (CN)
(72) Inventor: SUN, Minmin, Jiangsu 201210 (CN); LI, Yantao, Jiangsu 201210 (CN); WANG, Youtao, Jiangsu 201210 (CN); HAO, Ruidong, Jiangsu 201210 (CN); ZHONG, Yunpeng, Jiangsu 201210 (CN); YI, Qiaoyong, Jiangsu 201210 (CN)
(74) Representative: ZBM Patents - Zea, Barlocci & Markvardsen
(86) International application number: PCT/CN2022/105029
(87) International publication number: WO 2023/284700

(57) **Abstract**

A novel chimeric receptor composition, a recombinant vector, a cell, and an application thereof. The novel chimeric receptor composition includes a conventional chimeric antigen receptor (CAR), and a NKG2D chimeric receptor including a full-length sequence or a truncated fragment of NKG2D, DAP 10, and/or DAP 12, referred to as a SNR-armed CAR. The chimeric receptor composition enables a conventional CAR-T cell to express a NKG2D extracellular domain and an intracellular signal domain, expands a CAR-T antigen recognition spectrum, solves the tumor heterogeneity, achieves a lower level of cytokine release while enhancing the killing capability of CAR-T on tumor cells expressing target antigens, reduces the possibility of cytokine storm occurrence, and improves the CAR-T security.

## Description

### CROSS-REFERENCE TO RELATED APPLICATIONS

The present application claims priority to Chinese Patent Application No. 202110793058.6, filed with the China National Intellectual Property Administration on July 14, 2021 and entitled "NOVEL CHIMERIC RECEPTOR COMPOSITION, RECOMBINANT VECTOR, CELL, AND APPLICATION THEREOF", which is incorporated herein by reference in its entirety.

### TECHNICAL FIELD

The present disclosure relates to the technical field of biomedicine, and in particular to a novel chimeric receptor composition, recombinant vector, cell, and applications thereof.

### BACKGROUND

Conventional tumor treatment methods include surgery, radiotherapy, chemotherapy, targeted therapy, etc. In recent years, development of immunotherapy, especially immune checkpoint therapy such as CTLA-4 and PD-1/PD-L1 pathway inhibitors, and adoptive cell therapy such as CAR-T therapy, has brought profound changes to the field of tumor treatment. The adoptive cell therapy includes TIL, NK, TCR-T, CAR-T/NK/NKT/TIL/Mø, etc., among which, CD19-targeted CAR-T therapy has achieved excellent clinical efficacy in B-cell tumors, and in 2017, two CAR-T products were approved by the FDA for the treatment of B-cell leukemia or lymphoma.

Although significant progress has been made in the treatment of hematological tumors with CAR-T therapy, the incidence of side effects such ascytokine storm is relatively high in the treatment of hematological tumors. Most patients undergoing CAR-T therapy need auxiliary measures to mitigate side effects caused by the cytokine storm. One of the important reasons why CAR-T therapy has poor efficacy in the treatment of solid tumors is tumor heterogeneity. CAR-T cells have a weak capability of killing solid tumors and cannot completely eliminate tumor cells. In addition, compared with CAR-T therapy for hematological tumors, CAR-T therapy for solid tumors causes a higher incidence and a higher level of cytokine storm, and more severe side effects, and has a lower clinical benefit-risk ratio, thereby impeding the acceptance of CAR-T therapy in patients with clinical solid tumors.

Existing solutions typically employ dual targets to address the challenge of tumor heterogeneity. They intervene either during or prior to treatment using antibodies that symptomatically manage a cytokine storm or safety switches designed for CAR-T cells. In the event of a cytokine storm or excessive proliferation of CAR-T cells, these antibodies neutralize the cytokines or eliminate CAR-T cells, thereby mitigating the toxic side effects associated with CAR-T therapy..

NK cells express NKG2D on their surfaces and initiating downstream signals DAP10 and DAP12 upon recognizing their ligands such as MICA or MICB, thereby killing target cells. T cells also express NKG2D on their surfaces and express DAP10 but lack DAP12 intracellularly. Under normal circumstances, NKG2D on the surfaces of T cells cannot effectively activate intracellular signals after recognizing their ligands, resulting in an inability to eliminate target cells. The present disclosure intends to design expression of a NKG2D extracellular domain and an intracellular signal domain in conventional CAR-T cells to expand a CAR-T antigen recognition spectrum, while resolving the problem of tumor heterogeneity.

### SUMMARY OF THE INVENTION

In order to overcome the deficiencies in the prior art, the present disclosure provides a novel chimeric receptor composition or fusion protein, a recombinant vector, a cell, and use thereof, which expands the CAR-T antigen recognition spectrum while resolving the problem of tumor heterogeneity, reducing the possibility of a cytokine storm, and increasing the safety of CAR-T therapy.

To achieve the objectives, the following technical solutions are proposed in the present disclosure.

A first aspect of the present disclosure provides a novel chimeric receptor composition or fusion protein, including a chimeric antigen receptor and a NKG2D chimeric receptor comprising a full-length sequence or a truncated fragment of NKG2D, DAP10, and/or DAP12.

Further, the chimeric receptor composition or fusion protein comprises the chimeric antigen receptor and the NKG2D chimeric receptor, and the NKG2D chimeric receptor comprises sequences of a NKG2D extracellular domain and a DAP12 intracellular domain.

Further, the NKG2D chimeric receptor further comprises a costimulatory molecule selected from a group consisting of CD28, 4-1BB, DAP10, ICOS, OX40 and CD40.

Further, the chimeric antigen receptor comprises an extracellular recognition domain, an extracellular hinge domain, a transmembrane domain and an intracellular signal domain.

Further, the extracellular recognition domain comprises an antibody or a fragment thereof which recognizes a tumor-associated antigen or a tumor-specific antigen.

Further, the tumor-associated antigen or tumor-specific antigen is selected from a group consisting of CD19, BCMA, CD22, CD20, CD123, CD30, CD38, CD138, CD56, CD7, CLL-1, CD10, CD34, CS1, CD16, CD4, CD5, IL-1-RAP, ITGB7, k-IgG, TAC1, TRBC1, MUC1, NKG2D, PD-L1, CD133, CD177, LeY, CD70, ROR1, AFP, AXL, CD80, CD86, DLL3, DR5, FAP, LMP1, MAGE-A1, MAGE-A4, MG7, MUC16, PMEL, ROR2, VEGFR2, CD171, Claudin 18.2, Claudin 6, EphA2, ErbB, Fra, PSCA, cMet, IL13Ra2, EPCAM, EGFR, PSMA, EGFRvIII, GPC3, CEA, HER2, GD2, and Mesothelin.

Further, the hinge domain has a sequence derived from at least one of CD8α, CD28, 4-1BB, ICOS, OX40, CD40, CD80, and IgG; the transmembrane domain has a sequence derived from at least one of CD2, CD27, LFA-1 (CD11a/CD18), CD8α, CD28, 4-1BB, ICOS, OX40, CD40, CD80, CD3ζ, and CD3ε; and the intracellular signal domain has a sequence derived from at least one of a Toll-like receptor, CD2, CD27, LFA-1 (CD11a/CD18), CD8α, CD28, 4-1BB, ICOS, OX40, CD40, CD80, DAP10, DAP12, CD3ζ, and CD3ε.

Further, the chimeric receptor composition comprises a connecting peptide for connecting the chimeric antigen receptor with the chimeric receptor, and the connecting peptide is a self-cleaving polypeptide, i.e., a 2A peptide including F2A, P2A, T2A and E2A, and preferably, the connecting peptide is P2A having an amino acid sequence of SEQ ID NO. 9.

Further, an amino acid sequence of the chimeric receptor is one selected from SEQ ID NO. 1 to SEQ ID NO. 8, and preferably SEQ ID NO. 3 or SEQ ID NO. 6.

A second aspect of the present disclosure provides a nucleic acid encoding the chimeric receptor composition or fusion protein.

A third aspect of the present disclosure provides a vector including the nucleic acid.

A fourth aspect of the present disclosure provides a cell that expresses the chimeric receptor composition or fusion protein or comprises the nucleic acid or the vector.

Further, the cell is one selected from a group consisting of T cells, NK cells, denritic cells (DCs), and macrophages.

Further, the T cell is selected from an αβ T cell, a γδ T cell, or a NKT cell.

A fifth aspect of the present disclosure provides a biological agent for treatment of tumors, which comprises the cell as a main active ingredient.

A sixth aspect of the present disclosure provides a method for preparing the cell, including a step of transfecting the cell with the vector.

A seventh aspect of the present disclosure provides use of the chimeric receptor composition, nucleic acid, vector, or cell for preparation of an antineoplastic drug.

Compared with existing technology, the above technical solutions of the present disclosure have the following technical effects:
The chimeric receptor composition or fusion protein according to the present disclosure enables a conventional CAR-T cell to express a NKG2D extracellular domain and an intracellular signal domain, expands the CAR-T antigen recognition spectrum, solves tumor heterogeneity, achieves a lower level of cytokine release while enhancing the killing capability of CAR-T cells on tumor cells expressing target antigens, reduces the possibility of cytokine storm occurrence, and improves the safety of CAR-T therapy.

### BRIEF DESCRIPTION OF THE DRAWINGS

FIG. 1 shows the expression of mRNA of CLDN18.2 and NKG2D ligands in PDX models derived from gastric cancer (A), pancreatic cancer (B), and esophageal cancer (C), according to an embodiment of the present disclosure;
FIG. 2 shows the expression of proteins of CLDN18.2 and NKG2D ligands in gastric cancer tissue chips, according to an embodiment of the present disclosure;
FIG. 3 is a schematic structural diagram of a chimeric acceptor composition with sequence names of 21007 and 21067 to 21074, according to an embodiment of the present disclosure;
FIG. 4 shows the expression results of anti-CLDN18.2-CAR and NKG2D in constructed CAR-T cells, according to an embodiment of the present disclosure;
FIG. 5 shows high expression of CLDN18.2 in a constructed cell line 293-CLDN18.2, according to an embodiment of the present disclosure;
FIG. 6 shows NKG2D ligand MICA/B is relatively highly expressed in 293T, according to an embodiment of the present disclosure;
FIG. 7 shows the killing effect of SNR-armed CAR-T cells on cell line 293-CLDN18.2, according to an embodiment of the present disclosure;
FIG. 8 shows the proliferation multiple of CAR-T cells co-incubated with the cell line 293-CLDN18.2, according to an embodiment of the present disclosure;
FIG. 9 shows the killing effect of SNR-armed CAR-T cells on 293T cells, according to an embodiment of the present disclosure;
FIG. 10 shows that SNR-armed CAR-T cells have lower secretion levels of cytokines when killing target cells 293-CLDN18.2 according to an embodiment of the present disclosure, where FIG.10 A-D show the secretion levels of IL-2, IFN-γ, IL6, and TNF-α, respectively;
FIG. 11 shows that SNR-armed CAR-T cells release moderate levels of cytokines when killing target cells expressing MICA/B according to an embodiment of the present disclosure, where FIG.11 A-D show the secretion levels of IFN-γ, TNF-α, IL6, and IL-2, respectively;
FIG. 12 is a schematic diagram of SNR-armed CLDN18.2 CAR according to an embodiment of the present disclosure;
FIG. 13 is a diagram showing the result of expressing ULBP2/5/6 by A431 according to an embodiment of the present disclosure;
FIG. 14 is a diagram showing the results of killing *in vitro* and cytokine secretion experiments according to an embodiment of the present disclosure, where columns in FIG. C and FIG. D represent unT, 21047, 21326, 21327, 21328, and 21329, respectively, from left to right;
FIG. 15 shows the effect of SNR on CAR-T cell proliferation, memory phenotype, and exhaustion, according to an embodiment of the present disclosure;
FIG. 16 shows the experimental result of the study on the efficacy for CLDN18.2 homogeneous expression *in vivo* model according to an embodiment of the present disclosure;
FIG. 17 shows A431 rechallenge experiment according to an embodiment of the present disclosure;
FIG. 18 shows the efficacy experiment for the A431 tumor model according to an embodiment of the present disclosure, where columns in FIG. B represent PBS, UNT, 21047, 21327, 21328, and 21329, respectively, from left to right, and the PBS group coincides with the abscissa axis;
FIG. 19 shows the result of the efficacy experiment for the CLDN18.2 heterogeneous tumor model according to an embodiment of the present disclosure;
FIG. 20 shows a schematic diagram of SNR-armed EPCAM CAR and CD19 CAR according to an embodiment of the present disclosure;
FIG. 21 shows the expression results of anti-EPCAM-CAR, anti-CD19-CAR, and NKG2D in a constructed CAR-T cell according to an embodiment of the present disclosure;
FIG. 22 is a diagram of the experimental results of *in vitro* killing and IFN-γ secretion of EPCAM CAR-T according to an embodiment of the present disclosure; and
FIG. 23 is a diagram of the experimental results of *in vitro* killing and IFN-γ secretion of CD19 CAR-T according to an embodiment of the present disclosure.

### DETAILED DESCRIPTION

In the present disclosure, a chimeric receptor composition or fusion protein is constructed by adding a NKG2D chimeric receptor including a full-length sequence or a truncated fragment of NKG2D, DAP10, and/or DAP12 on the basis of an expression vector of a conventional chimeric antigen receptor. The vector is packaged into a lentivirus which is used to transduce cells for preparation of immune cells targeting CLDN18.2 while expressing NKG2D, thereby increasing the safety of the immune cell therapy.

"A full-length sequence of NKG2D, DAP10, and/or DAP12" means that a full-length sequence of NKG2D is necessarily comprised, and one or both of the full-length sequences of DAP10 and DAP12 are comprised.

In some embodiments, a truncated fragment of NKG2D chimeric receptor is comprised, and one or both of the full-length sequences of DAP10 and DAP12 are comprised. The truncated fragment of NKG2D chimeric receptor can be either a truncated fragment of an intracellular domain (ICD) or a truncated fragment of an extracellular domain (ECD) of NKG2D.

An embodiment of the present disclosure provides a novel chimeric receptor composition or fusion protein, comprising a chimeric antigen receptor and a NKG2D chimeric receptor comprising a full-length sequence or a truncated fragment of NKG2D, DAP10, and/or DAP12.

In a preferred embodiment of the present disclosure, the chimeric receptor composition or fusion protein comprises the chimeric antigen receptor and the NKG2D chimeric receptor, where the NKG2D chimeric receptor comprises sequences of a NKG2D extracellular domain and a DAP12 intracellular domain.

In a preferred embodiment of the present disclosure, the NKG2D chimeric receptor further comprises a costimulatory molecule selected from a group consisting of CD28, 4-1BB, DAP10, ICOS, OX40 and CD40.

In a preferred embodiment of the present disclosure, the chimeric antigen receptor comprises an extracellular recognition domain, an extracellular hinge domain, a transmembrane domain and an intracellular signal domain.

In a preferred embodiment of the present disclosure, the extracellular recognition domain comprises an antibody or a fragment thereof which recognizes a tumor-associated antigen or a tumor-specific antigen.

In a preferred embodiment of the present disclosure, the tumor-associated antigen or tumor-specific antigen is selected from a group consisting of CD19, BCMA, CD22, CD20, CD123, CD30, CD38, CD138, CD56, CD7, CLL-1, CD10, CD34, CS1, CD16, CD4, CD5, IL-1-RAP, ITGB7, k-IgG, TAC1, TRBC1, MUC1, NKG2D, PD-L1, CD133, CD177, LeY, CD70, ROR1, AFP, AXL, CD80, CD86, DLL3, DR5, FAP, LMP1, MAGE-A1, MAGE-A4, MG7, MUC16, PMEL, ROR2, VEGFR2, CD171, Claudin 18.2, Claudin 6, EphA2, ErbB, Fra, PSCA, cMet, IL13Ra2, EPCAM, EGFR, PSMA, EGFRvIII, GPC3, CEA, HER2, GD2, and Mesothelin, without limitation.

In a preferred embodiment of the present disclosure, the hinge domain has a sequence derived from at least one of CD8α, CD28, 4-1BB, ICOS, OX40, CD40, CD80, and IgG; the transmembrane domain has a sequence derived from at least one of CD2, CD27, LFA-1 (CD11a/CD18), CD8α, CD28, 4-1BB, ICOS, OX40, CD40, CD80, CD3ζ, and CD3ε; and the intracellular signal domain has a sequence derived from at least one of a Toll-like receptor, CD2, CD27, LFA-1 (CD11a/CD18), CD8α, CD28, 4-1BB, ICOS, OX40, CD40, CD80, DAP10, DAP12, CD3 ζ, and CD3ε.

In a preferred embodiment of the present disclosure, an amino acid sequence of the chimeric antigen receptor is SEQ ID NO. 19.

In a preferred embodiment of the present disclosure, the chimeric receptor composition or fusion protein further comprises a connecting peptide for connecting the chimeric antigen receptor with the chimeric receptor, and the connecting peptide is a self-cleaving polypeptide, i.e., a 2A peptide including F2A, P2A, T2A and E2A, and preferably, the connecting peptide is P2A having an amino acid sequence of SEQ ID NO. 9.

In a preferred embodiment of the present disclosure, an amino acid sequence of the chimeric receptor is one selected from SEQ ID NO. 1 to SEQ ID NO. 8, and preferably SEQ ID NO. 3 or SEQ ID NO. 6.

An embodiment of the present disclosure provides a nucleic acid encoding the chimeric receptor composition or fusion protein.

As used herein, nucleic acids include variants thereof with conservative substitutions (e.g., substitutions of degenerate codons) and complementary sequences, as well as variants optimized by codons for more efficient expression in a desired host cell. The nucleic acid is typically RNA or DNA, including a gene, a cDNA molecule, an mRNA molecule, and a fragment thereof such as an oligonucleotide. The nucleic acid molecule may be single-stranded or double-stranded, but is preferably double-stranded DNA. When forming a functional relationship with another nucleic acid, the nucleic acid is "effectively connected". For example, if a promoter or an enhancer affects transcription of a coding sequence, then the promoter or enhancer is effectively connected to the coding sequence. When connected to the vector, the nucleic acid molecule is preferably DNA nucleic acid.

An embodiment of the present disclosure provides a vector comprising the nucleic acid.

The term "vector" refers to a nucleic acid delivery vehicle into which a polynucleotide can be inserted. When a vector enables an inserted polynucleotide-encoded protein to be expressed, the vector is referred to as an expression vector. The vector can enter a host cell via transformation, transduction, or transfection, so that an element of a genetic material carried by the vector is expressed in the host cell. The vector is well-known to a person skilled in the art and includes, but is not limited to, a plasmid, CRISPR/CAS plasmid, a phagemid, a cosmid, an artificial chromosome (for example, a yeast artificial chromosome (YAC), a bacterial artificial chromosome (BAC), or a P1 derived artificial chromosome (PAC)), a phage (λ phage or M13 phage), and an animal virus. An animal virus that can serve as the vector includes, but is not limited to, retrovirus, lentivirus, adenovirus, adeno-associated virus, herpesvirus (for example, herpes simplex virus), poxvirus, baculovirus, papillomavirus, and papillomavirus (for example, SV40). In some embodiments, the vector in the present disclosure includes a regulatory element commonly used in genetic engineering, for example, the enhancer, the promoter, an internal ribosome entry site (IRES) and another expression control element (for example, a transcription termination signal, or a polyadenylation signal and a polyU sequence).

An embodiment of the present disclosure provides a cell that expresses the chimeric receptor composition or fusion protein, or comprises the nucleic acid or the vector.

The cell is preferably an immuno-killer cell.

In a preferred embodiment of the present disclosure, the cell is one selected from a group consisting of T cells, NK cells, denritic cells (DCs), and macrophages.

The T cell in the present disclosure may include subcategories well-known in the art, for example, one or more of a helper T cell, a cytotoxic T cell, a memory T cell, a regulatory T cell, a MAIT cell, a NKT cell, and a γδT cell. In a preferred embodiment of the present disclosure, the T cell is an αβ T cell, a γδ T cell, or a NKT cell.

An embodiment of the present disclosure provides a biological agent for treating a tumor, which comprises the cell as a main active ingredient, and preferably, the biological agent further comprises a pharmaceutically acceptable vector, a diluent, and/or an excipient.

As used herein, "a pharmaceutically acceptable vector, a diluent, and/or an excipient" includes any material that allows a component to remain biologically active when combined with the active component and that does not react with an immune system of the subject. An example includes, but is not limited to, any one of a standard drug vector (for example, a phosphate buffered saline solution, water, an emulsion (for example, an oil or water emulsion)) and various types of wetting agents. Exemplary diluents for aerosol or parenteral administration are phosphate buffered saline (PBS) or normal saline (0.9%). A composition containing such vector is prepared in a well-known conventional method (refer to, for example, Remington's Pharmaceutical Sciences, 18th edition, A. Gennaro, ed., Mack Publishing Co., Easton, PA, 1990; and Remington, The Science and Practice of Pharmacy, 21st edition, Mack Publishing, 2005).

An embodiment of the present disclosure provides a method for preparing the cell, including a step of transfecting the cell with the vector.

An embodiment of the present disclosure provides use of the chimeric receptor composition, the nucleic acid, the vector, or the cell for preparation of an antineoplastic drug.

In a preferred embodiment, the antineoplastic drug is used for the treatment of a heterogeneous tumor.

In a preferred embodiment of the present disclosure, the heterogeneous tumor includes at least NKG2DL-positive tumor cells and tumor cells targeted by the chimeric antigen receptor.

The present disclosure is described in detail via specific examples and drawings below, to better understand the present disclosure, but the following examples impose no limitation on the scope of the present disclosure.

In the examples, unless otherwise specified, the method is a conventional method; and unless otherwise specified, a used reagent is a conventional commercially available reagent or a reagent prepared in the conventional method.

### Example 1: Detection of Expression of CLDN18.2 and NKG2D Ligands in Gastric Cancer Tissues

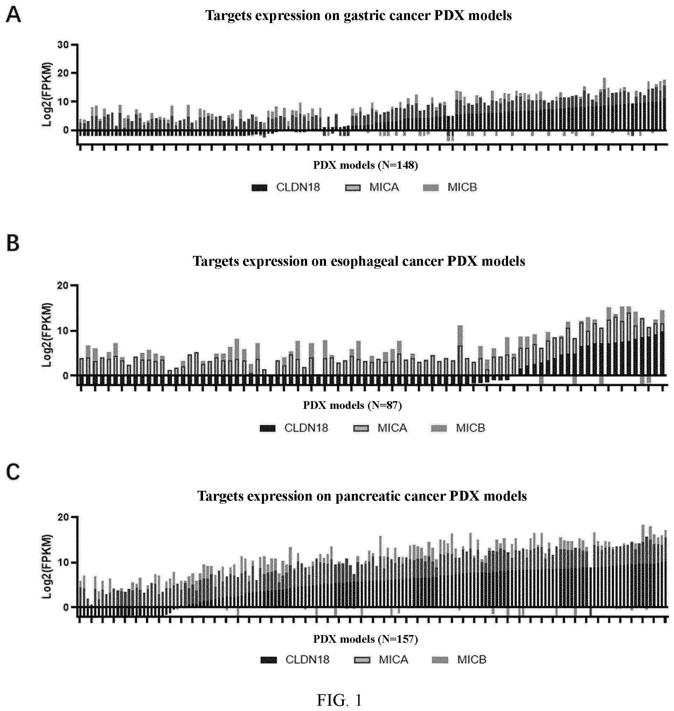

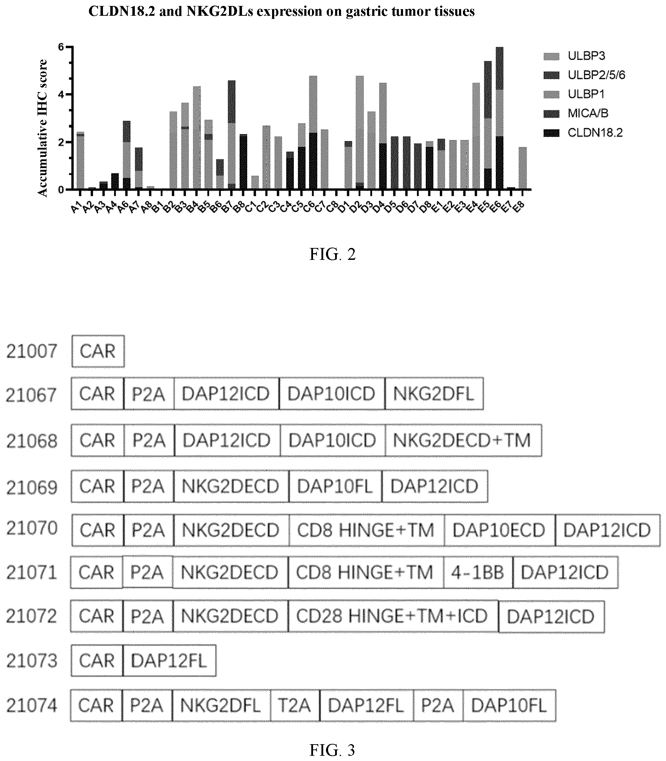

The inventors first analyzed expression of CLDN18.2 and NKG2DL mRNA in gastric, pancreatic, and esophageal cancer-derived PDX tissues by using the PDX database (i.e. CrownBio) and obtained results as shown in FIG. 1, in which positive rates of CLDN18.2 in the gastric, pancreatic and esophageal cancer-derived PDX tissues were 63%, 82% and 26%, and most tissues showed expression of at least one NKG2D ligand or CLDN18.2. Similarly, the inventors detected expression of CLDN18.2 and NKG2D ligands (i.e. MICA, MICB, and ULBP1 to 6) in gastric cancer tissue chips (commercially available from Bioaitech) and obtained results consistent with those from the PDX database, with most tissues expressing at least one NKG2D ligand or CLDN18.2 (FIG. 2). The above results indicate that the use of targeting NKG2DL may resolve the problem of tumor heterogeneity when using a single-targeting product such as CLDN18.2.

### Example 2: Design of Synthetic Natural Killer Receptor (SNR)

This example describes a novel chimeric receptor composition (hereinafter referred to as Dual CAR, also known as SNR, or SNR-armed CAR molecule, with a corresponding CAR-T cell being referred to as Dual CAR-T or SNR-armed CAR-T), which includes anti-CLDN18.2-CAR with an amino acid sequence of SEQ ID NO. 1 (i.e. conventional second-generation Claudin18.2 CAR molecule, hereinafter referred to as 21007) in the Chinese patent application published under CN113621073A on November 09, 2021 and chimeric acceptor sequences denoted as SEQ ID NO. 1 to SEQ ID NO. 8 (with structural sequences as shown in FIG. 3). Specific amino acid sequences correspond to sequences Nos. 21067 to 21074 in Table 1 below.

**Table 1 Sequence Information of Anti-CLDN18.2-CAR and Chimeric Receptor Compositions**

| **Sequence/Plasmid name** | **Amino acid sequence** |
|---|---|
| 21007 | SEQ ID NO. 1 in the Chinese patent application published under CN113621073A on November 09, 2021 is referred to. |
| 21067 | YFLGRLVPRGRGAAEAATRKQRITETESPYQELQGQRS DVYSDLNTQRPYYKLCARPRRSPAQEDGKVYINMPGR GMGWIRGRRSRHSWEMSEFHNYNLDLKKSDFSTRWQ KQRCPVVKSKCRENASPFFFCCFIAVAMGIRFIIMVTIWS AVFLNSLFNQEVQIPLTESYCGPCPKNWICYKNNCYQFF DESKNWYESQASCMSQNASLLKVYSKEDQDLLKLVKS YHWMGLVHIPTNGSWQWEDGSILSPNLLTIIEMQKGDC ALYASSFKGYIENCSTPNTYICMQRTV (SEQ ID NO. 1) |
| 21068 | YFLGRLVPRGRGAAEAATRKQRITETESPYQELQGQRS DVYSDLNTQRPYYKLCARPRRSPAQEDGKVYINMPGR GPFFFCCFIAVAMGIRFIIMVTIWSAVFLNSLFNQEVQIPL TESYCGPCPKNWICYKNNCYQFFDESKNWYESQASCM SQNASLLKVYSKEDQDLLKLVKSYHWMGLVHIPTNGS WQWEDGSILSPNLLTIIEMQKGDCALYASSFKGYIENCS TPNTYICMQRTV (SEQ ID NO. 2) |
| 21069 | MIHLGHILFLLLLPVAAAFNQEVQIPLTESYCGPCPKNW ICYKNNCYQFFDESKNWYESQASCMSQNASLLKVYSK EDQDLLKLVKSYHWMGLVHIPTNGSWQWEDGSILSPN |
| | LLTIIEMQKGDCALYASSFKGYIENCSTPNTYICMQRTV QTTPGERSSLPAFYPGTSGSCSGCGSLSLPLLAGLVAAD AVASLLIVGAVFLCARPRRSPAQEDGKVYINMPGRGYFL GRLVPRGRGAAEAATRKQRITETESPYQELQGQRSDVY SDLNTQRPYYK (SEQ ID NO. 3) |
| 21070 | MALPVTALLLPLALLLHAARPFNQEVQIPLTESYCGPCP KNWICYKNNCYQFFDESKNWYESQASCMSQNASLLK VYSKEDQDLLKLVKSYHWMGLVHIPTNGSWQWEDGSI LSPNLLTIIEMQKGDCALYASSFKGYIENCSTPNTYICMQ RTVTTTPAPRPPTPAPTIASQPLSLRPEACRPAAGGAVHT RGLDFACDIYIWAPLAGTCGVLLLSLVITLYCLCARPRR SPAQEDGKVYINMPGRGYFLGRLVPRGRGAAEAATRK QRITETESPYQELQGQRSDVYSDLNTQRPYYK (SEQ ID NO. 4) |
| 21071 | MALPVTALLLPLALLLHAARPFNQEVQIPLTESYCGPCP KNWICYKNNCYQFFDESKNWYESQASCMSQNASLLK VYSKEDQDLLKLVKSYHWMGLVHIPTNGSWQWEDGSI LSPNLLTIIEMQKGDCALYASSFKGYIENCSTPNTYICMQ RTVTTTPAPRPPTPAPTIASQPLSLRPEACRPAAGGAVHT RGLDFACDIYIWAPLAGTCGVLLLSLVITLYCKRGRKKL LYIFKQPFMRPVQTTQEEDGCSCRFPEEEEGGCELYFLG RLVPRGRGAAEAATRKQRITETESPYQELQGQRSDVYS DLNTQRPYYK (SEQ ID NO. 5) |
| 21072 | MALPVTALLLPLALLLHAARPFNQEVQIPLTESYCGPCP KNWICYKNNCYQFFDESKNWYESQASCMSQNASLLK VYSKEDQDLLKLVKSYHWMGLVHIPTNGSWQWEDGSI LSPNLLTIIEMQKGDCALYASSFKGYIENCSTPNTYICMQ RTVIEVMYPPPYLDNEKSNGTIIHVKGKHLCPSPLFPGP SKPFWVLVVVGGVLACYSLLVTVAFIIFWVRSKRSRLL HSDYMNMTPRRPGPTRKHYQPYAPPRDFAAYRSYFLG RLVPRGRGAAEAATRKQRITETESPYQELQGQRSDVYS DLNTQRPYYK (SEQ ID NO. 6) |
| 21073 | MGGLEPCSRLLLLPLLLAVSGLRPVQAQAQSDCSCSTV SPGVLAGIVMGDLVLTVLIALAVYFLGRLVPRGRGAAE AATRKQRITETESPYQELQGQRSDVYSDLNTQRPYYK (SEQ ID NO. 7) |
| 21074 | MALPVTALLLPLALLLHAARPMGWIRGRRSRHSWEMS EFHNYNLDLKKSDFSTRWQKQRCPVVKSKCRENASPF FFCCFIAVAMGIRFIIMVTIWSAVFLNSLFNQEVQIPLTES |
| | YCGPCPKNWICYKNNCYQFFDESKNWYESQASCMSQ NASLLKVYSKEDQDLLKLVKSYHWMGLVHIPTNGSWQ WEDGSILSPNLLTIIEMQKGDCALYASSFKGYIENCSTPN TYICMQRTVGSGEGRGSLLTCGDVEENPGPMGGLEPCS RLLLLPLLLAVSGLRPVQAQAQSDCSCSTVSPGVLAGIV MGDLVLTVLIALAVYFLGRLVPRGRGAAEAATRKQRIT ETESPYQELQGQRSDVYSDLNTQRPYYKGSGATNFSLL KQAGDVEENPGPMIHLGHILFLLLLPVAAAQTTPGERSS LPAFYPGTSGSCSGCGSLSLPLLAGLVAADAVASLLIVG AVFLCARPRRSPAQEDGKVYINMPGRG (SEQ ID NO. 8) |

As shown in FIG. 3, except for the chimeric antigen receptor 21007, the above chimeric receptor compositions also include the following sequence information:
(1) P2A having an amino acid sequence of:
   GSGATNFSLLKQAGDVEENPGP (SEQ ID NO. 9);
(2) DAP12ICD (intracellular domain) having an amino acid sequence of:
   YFLGRLVPRGRGAAEAATRKQRITETESPYQELQGQRSDVYSDLNTQRPYYK (SEQ ID NO. 10);
(3) DAP10ICD having an amino acid sequence of:
   LCARPRRSPAQEDGKVYINMPGRG (SEQ ID NO. 11);
(4) NKG2DFL (full-length sequence) having an amino acid sequence of: 
MGWIRGRRSRHSWEMSEFHNYNLDLKKSDFSTRWQKQRCPVVKSKCRENASPFFFCCFIA
VAMGIRFIIMVTIWSAVFLNSLFNQEVQIPLTESYCGPCPKNWICYKNNCYQFFDESKNWYE
SQASCMSQNASLLKVYSKEDQDLLKLVKSYHWMGLVHIPTNGSWQWEDGSILSPNLLTIIE
MQKGDCALYASSFKGYIENCSTPNTYICMQRTV (SEQ ID NO. 12);
(5) CD8 hinge (hinge domain) having an amino acid sequence of:
   TTTPAPRPPTPAPTIASQPLSLRPEACRPAAGGAVHTRGLDFACD (SEQ ID NO. 13);
(6) CD8 TM (transmembrane domain) having an amino acid sequence of:
   IYIWAPLAGTCGVLLLSLVITLYC (SEQ ID NO. 14);
(7) DAP10FL having an amino acid sequence of: 
MIHLGHILFLLLLPVAAAQTTPGERSSLPAFYPGTSGSCSGCGSLSLPLLAGLVAADAVASLLI
VGAVFLCARPRRSPAQEDGKVYINMPGRG (SEQ ID NO. 15);
(8) DAP12FL having an amino acid sequence of: MGGLEPCSRLLLLPLLLAVSGLRPVQAQAQSDCSCSTVSPGVLAGIVMGDLVLTVLIALAVY 
FLGRLVPRGRGAAEAATRKQRITETESPYQELQGQRSDVYSDLNTQRP YYK (SEQ ID NO.
16);
(9) DAP12ECD + TM (extracellular domain and transmembrane domain) having an amino acid sequence of:
   LRPVQAQAQSDCSCSTVSPGVLAGIVMGDLVLTVLIALAV (SEQ ID NO. 17);
(10) T2A having an amino acid sequence of:
   GSGEGRGSLLTCGDVEENPGP (SEQ ID NO. 18).

### Example 3: Plasmid Construction and Viral Packaging of Synthetic Natural Killer Receptor (SNR)-Armed CAR Molecules

This example describes a SNR-armed CAR-T cell expressing the above chimeric receptor, and the construction method of the SNR-armed CAR-T cell comprises steps of:
1. Construction of Expression Vector: The SNR-armed CAR molecule and lentiviral vector skeleton were constructed by CRO using whole gene synthesis technology. A SNR-armed CAR fragment was cloned to the lentiviral vector via conventional molecular cloning, and names of the constructed plasmids are shown in Table 1 above. Each plasmid was proven to be constructed successfully by verifying a full sequence of an inserted fragment.
2. Lentiviral Packaging and Titer Measurement
   Day 1:
      1) 5×10⁶ 293T cells were inoculated in a T75 culture flask and a culture volume was 20 mL.
   Day 2:
      2) Before the viruses were packaged, it was confirmed that confluency of 293T cells was about 70% to 80%, and equal volumes of fluids were exchanged.
      3) Preparation and Transfection of Transfection Complex:
         Tube A and Tube B were configured respectively, the system is shown below, and Tube A and Tube B were inverted or oscillated at a low speed for uniform mixing;
         Tube A: Opti-MEM (2 mL) + Lipo3000 (55 µL);
         Tube B: Opti-MEM (2 mL) + P3000 (46 µL) + helper plasmid (18 µg) + main plasmid of SNR-armed CAR (6 µg);
         the culture medium from Tube A was added to Tube B, mixed well by shaking, and incubated at room temperature for 15 minutes; and
         the flask in which the liquid had been replaced in step 3 was turned over, so that the medium was on the other side of the flask; and the mixture of the Tube A and Tube B was added, shaken gently and mixed well, and then the flask was slowly turned back to the front, and then incubation continued for 48 hours.
   Day 4:
      4) After 2 days of incubation following transfection, the supernatant was collected and centrifuged at 500g for 10 minutes, the supernatant was filtered into a 50 mL centrifuge tube via a 0.45µm filter, the centrifuge tube was sealed with the parafilm, and centrifuged at 10000g at 4°C overnight, and a white virus precipitate was observed; and after the supernatant was discarded, the centrifuge tube was inverted until the supernatant was drained, the precipitate was dissolved in 200 µL of AIM-V medium, 2 µL of a mixture was taken to measure the titer according to the subsequent step, and the remaining mixture was stored at -80°C.
      5) 2 µL of resuspended viral supernatant was added to 198 µL of 1640 medium to dilute the viruses, then 2 µL, 10 µL, and 50 µL of diluted viruses were added to 3 wells, each of which contained 2×10⁵ Jurkat T cells on a 24-well plate, and polybrene at a final concentration of 5 µg/mL was added to enhance virus infection.
   Day 6:
      6) Anti-VHH-FITC antibody was used to measure titer of the viruses, and the titer ranged from 2.5×10⁷ to 1.2×10⁸, which could meet a requirement for a conventional *in vitro* CAR-T construction experiment.
3. Construction of CAR-T Cell Armed with Synthetic Natural Killer Receptor (SNR)

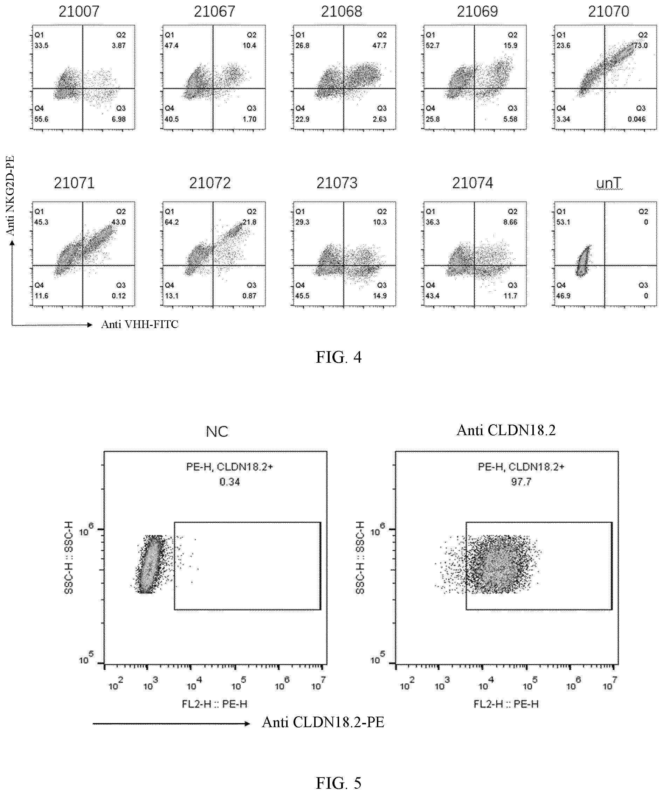

The packaged lentivirus was used to infect T cells to detect expression of anti-CLDN18.2-CAR and NKG2D in the T cells, obtaining results shown in FIG. 4.

It can be seen from FIG. 4 that anti-CLDN18.2-CAR-T cells showed a lower NKG2D expression level, and the SNR-armed CAR-T cells had a higher NKG2D expression level than the anti-CLDN18.2-CAR-T cells, while expressing anti-CLDN18.2-CAR normally. This indicates that the NKG2D chimeric receptor was successfully expressed on the surfaces of SNR-armed CAR-T cells.

### Example 4

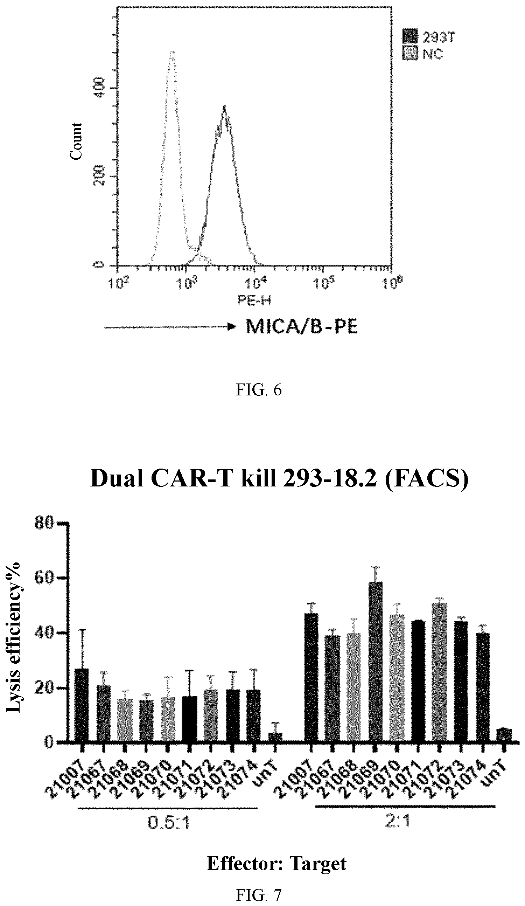

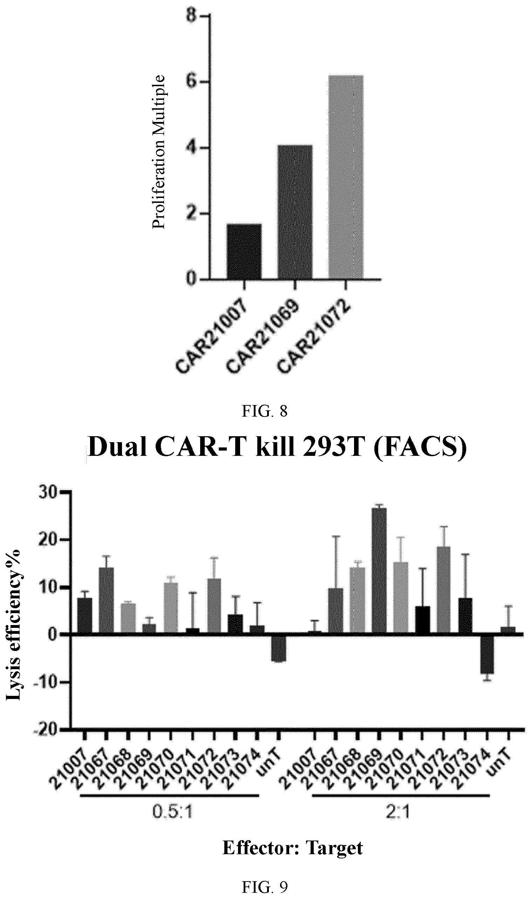

This example intended to verify the killing effect of SNR-armed CAR-T cells on target cells, and specific steps and results are as follows:
(1) Cell line 293-CLDN18.2 overexpressing the CLDN18.2 antigen was constructed on the basis of 293 cells, and the cell line highly expressed CLDN18.2 (as shown in FIG. 5).
(2) Detection of Expression of NKG2D Ligand MICA/B of the Cell Line: An expression level of MICA/B in the 239T cell line was determined, and the expression level of MICA/B was higher in the 293T cell line (as shown in FIG. 6).
(3) CAR-T cells and 293-CLDN18.2 were mixed in effect/target ratios of 2:1 and 0.5:1, the mixture was incubated for 5 hours, the killing effect of CAR-T on target cells was detected via Annexin V flow cytometry, anti-CLDN18.2-CAR-T cells and SNR-armed CAR-T cells had a stronger killing effect on 293-CLDN18.2 than unT (untransduced T) cells, and 21069 and 21072 in the effect/target ratio of 2:1 had a stronger killing effect than the conventional CAR-T cells (21007) (as shown in FIG. 7). In addition, 293-CLDN18.2 was co-incubated with CAR-T cells, and the proliferation multiple of SNR-armed CAR-T cells 21069 and 21072 were significantly higher than that of the conventional CAR-T cells (21007) (as shown in FIG. 8).

CAR-T cells and 293T cells were mixed in effect/target ratios of 2:1 and 0.5:1, the mixtures were incubated for 5 hours, the killing effect of CAR-T cells on target cells was detected via Annexin V flow cytometry, anti-CLDN18.2-CAR-T cells and SNR-armed CAR-T cells had a stronger killing effect on target cells expressing MICA/B than unT (untransduced T) cells, and 21069 and 21072 in the effect/target ratio of 2:1 had a stronger killing effect than the conventional CAR-T cells (as shown in FIG. 9).

### Example 5

This example verifies that SNR-armed CAR-T cells showed lower secretion levels of cytokines when killing target cells 293-CLDN18.2, and specific steps and results are as follows:

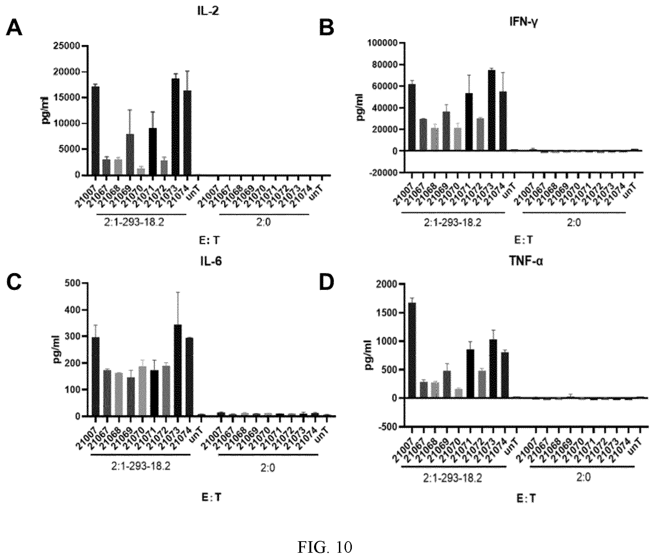

The cell line 293-CLDN18.2 constructed in Example 4 was used; and CAR-T cells and 293-CLDN18.2 were mixed in an effect/target ratio of 2:1, the mixture was incubated for 24 hours, and cytokine release of CAR-T was detected via flow cytometry. Biolegend's multi-cytokine detection kit was used, 15 µL of a supernatant of incubated effector cells and target cells were added per sample into a new V-type 96-well plate, a diluted mixture of 15 µL of magnetic beads per sample and 15 µL of assay buffer per sample were added, and the V-type 96-well plate was sealed with a plate sealing membrane, oscillated at 500 rpm at room temperature for 2 hours, and centrifuged at 250g for 5 minutes. The supernatant was discarded, 200 µL of wash buffer was added per sample, and the mixture was centrifuged at 250g for 5 minutes. The supernatant was discarded, 15 µL of detection antibody was added per sample, and the mixture was oscillated at 500 rpm at room temperature for 1 hour, and 15 µL of SA-PE was added per sample, the mixture was oscillated at 500 rpm at room temperature for 30 minutes, and centrifuged at 250g for 5 minutes. The supernatant was discarded, 200 µL of wash buffer was added per sample, and the mixture was centrifuged at 250g for 5 minutes. The supernatant was discarded, 150 µL of wash buffer was added per sample, and the mixture was tested via an instrument. The results showed that when anti-CLDN18.2-CAR-T and SNR-armed CAR-T cells killed 293-CLDN18.2 target cells, 21067, 21068, 21069, 21070, 21072 and other SNR-armed CAR-T cells had significantly lower secretion levels of IFN-γ, IL2, IL6, and TNF-α (as shown in FIG. 10).

### Example 6

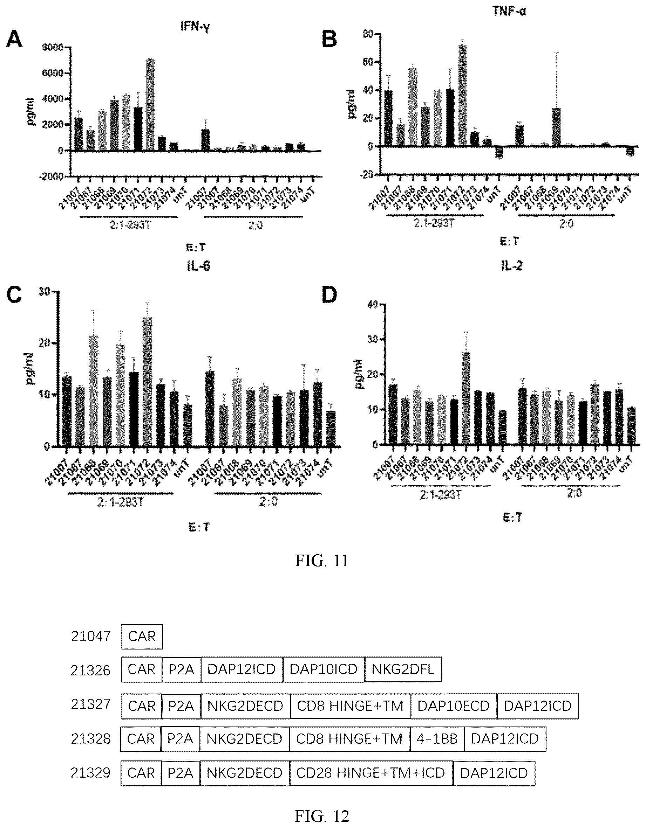

This example verifies that SNR-armed CAR-T cells release moderate levels of cytokines when killing target cells expressing MICA/B, and specific steps and results are as follows:
CAR-T cells and 293T were mixed in an effect/target ratio of 2:1, the mixture was incubated for 24 hours, and cytokine release of CAR-T was detected via flow cytometry. When anti-CLDN18.2-CAR-T cells and SNR-armed CAR-T cells killed 293T target cells, 21068, 21069, 21070, 21072 and other SNR-armed CAR-T cells had a moderate secretion level of IFN-γ and lower secretion levels of TNF-α, IL2, and IL6 (as shown in FIG. 11).

### Example 7: Construction of SNR-armed CLDN18.2 CAR-T Cells

Based on the above results, the inventors selected SNR structures within molecules of 21067, 21070, 21071, and 21072 for further verification. The inventors constructed the above SNRs into the second-generation CAR molecule 21047 to form new CAR molecules 21326, 21327, 21328, and 21329. Structures and sequences of the new CAR molecules are shown in FIG. 12 and the table below. Referring to the method in Example 2, the inventors constructed plasmids, lentiviruses, and CAR-T cells with the above molecules.

| **Sequence/Plasmid name** | **Amino acid sequence** |
|---|---|
| 21047 | MLLLVTSLLLCELPHPAFLLIPQVQLVESGGGLVQPGGSLR LSCAASGSIFNIPVMGWYRQAPGKQRELVAGISTGGTTNY GDSVKGRFTISRDNAKNTVYLQMNSLKPEDTAVYYCNVL VVSGIGSTLEVWGQGTLVTVSSIEVMYPPPYLDNEKSNGT IIHVKGKHLCPSPLFPGPSKPFWVLVVVGGVLACYSLLVT VAFIIFWVRSKRSRLLHSDYMNMTPRRPGPTRKHYQPYAP PRDFAAYRSRVKFSRSADAPAYQQGQNQLYNELNLGRREE YDVLDKRRGRDPEMGGKPRRKNPQEGLYNELQKDKMAE AYSEIGMKGERRRGKGHDGLYQGLSTATKDTYDALHMQ ALPPR (SEQ ID NO. 19) |
| 21326 | YFLGRLVPRGRGAAEAATRKQRITETESPYQELQGQRSDV YSDLNTQRPYYKLCARPRRSPAQEDGKVYINMPGRGMG WIRGRRSRHSWEMSEFHNYNLDLKKSDFSTRWQKQRCPV VKSKCRENASPFFFCCFIAVAMGIRFIIMVTIWSAVFLNSLF NQEVQIPLTESYCGPCPKNWICYKNNCYQFFDESKNWYES QASCMSQNASLLKVYSKEDQDLLKLVKSYHWMGLVHIPT NGSWQWEDGSILSPNLLTIIEMQKGDCALYASSFKGYIENC STPNTYICMQRTV (SEQ ID NO. 20) |
| 21327 | MALPVTALLLPLALLLHAARPFNQEVQIPLTESYCGPCPKN WICYKNNCYQFFDESKNWYESQASCMSQNASLLKVYSK EDQDLLKLVKSYHWMGLVHIPTNGSWQWEDGSILSPNLL TIIEMQKGDCALYASSFKGYIENCSTPNTYICMQRTVTTTP APRPPTPAPTIASQPLSLRPEACRPAAGGAVHTRGLDFACDI YIWAPLAGTCGVLLLSLVITLYCLCARPRRSPAQEDGKVYI |
| | NMPGRGYFLGRLVPRGRGAAEAATRKQRITETESPYQELQ GQRSDVYSDLNTQRPYYK (SEQ ID NO. 21) |
| 21328 | MALPVTALLLPLALLLHAARPFNQEVQIPLTESYCGPCPKN WICYKNNCYQFFDESKNWYESQASCMSQNASLLKVYSK EDQDLLKLVKSYHWMGLVHIPTNGSWQWEDGSILSPNLL TIIEMQKGDCALYASSFKGYIENCSTPNTYICMQRTVTTTP APRPPTPAPTIASQPLSLRPEACRPAAGGAVHTRGLDFACDI YIWAPLAGTCGVLLLSLVITLYCKRGRKKLLYIFKQPFMRP VQTTQEEDGCSCRFPEEEEGGCELYFLGRLVPRGRGAAEA ATRKQRITETESPYQELQGQRSDVYSDLNTQRPYYK (SEQ ID NO. 22) |
| 21329 | MALPVTALLLPLALLLHAARPFNQEVQIPLTESYCGPCPKN WICYKNNCYQFFDESKNWYESQASCMSQNASLLKVYSK EDQDLLKLVKSYHWMGLVHIPTNGSWQWEDGSILSPNLL TIIEMQKGDCALYASSFKGYIENCSTPNTYICMQRTVIEVM YPPPYLDNEKSNGTIIHVKGKHLCPSPLFPGPSKPFWVLVV VGGVLACYSLLVTVAFIIFWVRSKRSRLLHSDYMNMTPRR PGPTRKHYQPYAPPRDFAAYRSYFLGRLVPRGRGAAEAAT RKQRITETESPYQELQGQRSDVYSDLNTQRPYYK (SEQ ID NO. 23) |

### Example 8: Detection of Target Cell-Killing Capability of SNR-armed CLDN18.2 CAR-T Cells

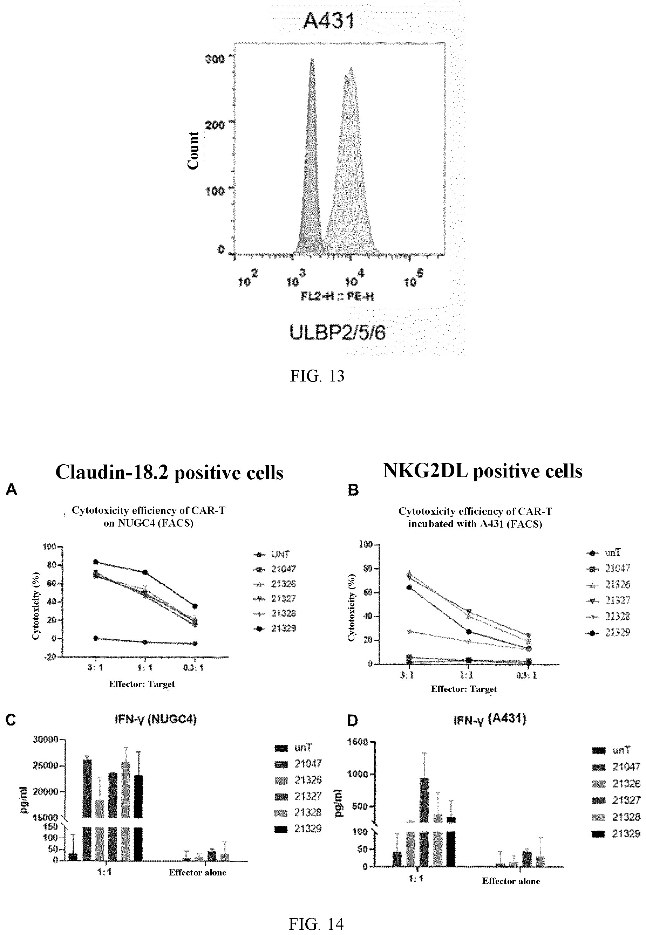

To verify whether the SNR can successfully kill NKG2DLs-positive cells, the inventors used ULBPs-positive tumor cells A431 to detect the killing capability of SNR. Specific steps and results are as follows:
(1) Detection of Expression of NKG2D Ligand ULBP2/5/6 of the Cell Line: An expression level of NKG2D ligand in the A431 cell line was determined, and the expression level of ULBP2/5/6 was higher in the A431 cell line (as shown in FIG. 13).
(2) CAR-T cells, NUGC4, and A431 were mixed in effect/target ratios of 3:1, 1:1, and 0.3:1, the mixtures were incubated for 5 hours, and the killing effect of CAR-T cells on target cells was detected via Annexin V flow cytometry. The results are shown in the figure. 21327 and 21329 in a high effect/target ratio had higher efficiency of killing NUGC4 than 21047. In addition, 21047 could not kill A431, while 21326 to 21329 could effectively kill A431. Multi-cytokine detection showed that IFN-γ secretion level of 21047 was higher than those of 21326 to 21329 when NUGC4 was being killed, but the IFN-γ secretion levels of 21327 to 21329 when A431 was being killed were generally lower than those when NUGC4 was being killed (as shown in FIG. 14). The results showed that all 4 SNR designs could target and kill CLAUDIN18.2 and NKG2DLs-positive cells, and implement a lower cytokine secretion level.

### Example 9: SNR Increases Proliferation Capacity and Memory Level of CAR-T Cells While Reducing Level of Exhaustion

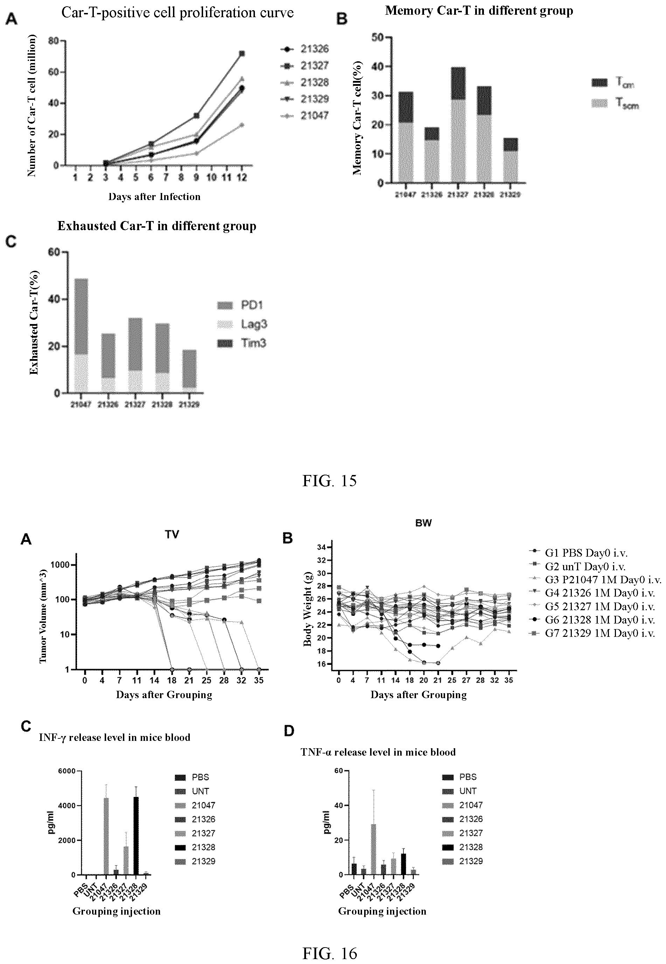

The inventors further analyzed an *in vitro* effect of SNR on CAR-T cells. The results are shown in FIG. 15. Compared with 21047, a proliferation rate of each molecule was significantly increased, while expression of each of the exhaustion markers (PD1, LAG3, and TIM3) was significantly reduced. Further analysis of a proportion of memory cells in the memory center showed that a proportion of Tscm cells for molecule 21327 was significantly higher than those for other molecules. The above advantages may be related to functions of a signal domain used in SNR.

### Example 10: Study on the Efficacy via CLDN18.2 Homogeneous Expression in vivo Model

To study *in vivo* efficacy of the above molecules, the inventors constructed a subcutaneously transplanted tumor model of NUGC4 (CLDN18.2+, NKG2DLs-) and each CAR-T was administered when a volume of the tumor reached 100 mm³. Study results showed that compared with 21047, 21327 could eliminate a tumor tissue about 10 days earlier, indicating better efficacy. In addition, efficacy of 21326 and 21329 was lower than that of 21047. Efficacy of 21328 could not be concluded due to premature death of mice (A in FIG. 16). The above results showed that compared with the conventional second-generation CAR-T 21047, molecule 21327 had a stronger capability of eliminating tumors with homogeneous expression of CLDN18.2.

During further weight analysis of the mice, weight of the mice receiving molecules 21328 decreased significantly during a study period, and death of mice occurred, indicating that the molecules had significant *in vivo* toxicity. One mouse also experienced weight loss during the study of molecule 21047, but no death of mice occurred. In addition, molecules 21326, 21327, and 21329 showed no significant toxicity throughout the study period (B in FIG. 16). Similarly, the inventors further analyzed cytokine expression levels in the blood of mice, and the results showed that molecules 21326, 21327, and 21329 had lower *in vivo* secretion levels of IFN-gamma (C in FIG. 16) and TNF-alpha (D in FIG. 16), and such results were consistent with safety results.

The above results showed that the molecule 21327 had better efficacy and lower toxicity than the conventional second-generation CAR-T. This indicated that the design of SNR effectively improved effectiveness and safety of CAR-T.

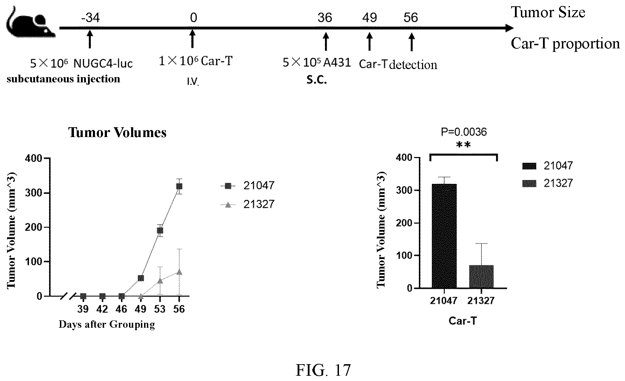

A capability of the CAR-T cells to prevent CLDN18.2-negative recurrence after CLDN18.2-positive tumors have been eliminated was further studied. The inventors used A431 cells (CLDN18.2-, NKG2DLs+) to conduct a rechallenge experiment on mice in groups 21047 and 21327 after tumor elimination. Study results showed that compared to 21047, the molecules 21327 could significantly inhibit the growth of A431 tumor (FIG. 17). The above results indicated that the addition of SNR can inhibit recurrence of CLDN18.2-negative tumor.

### Example 11: Efficacy Experiment of SNR via NKG2DL+ Tumor Model

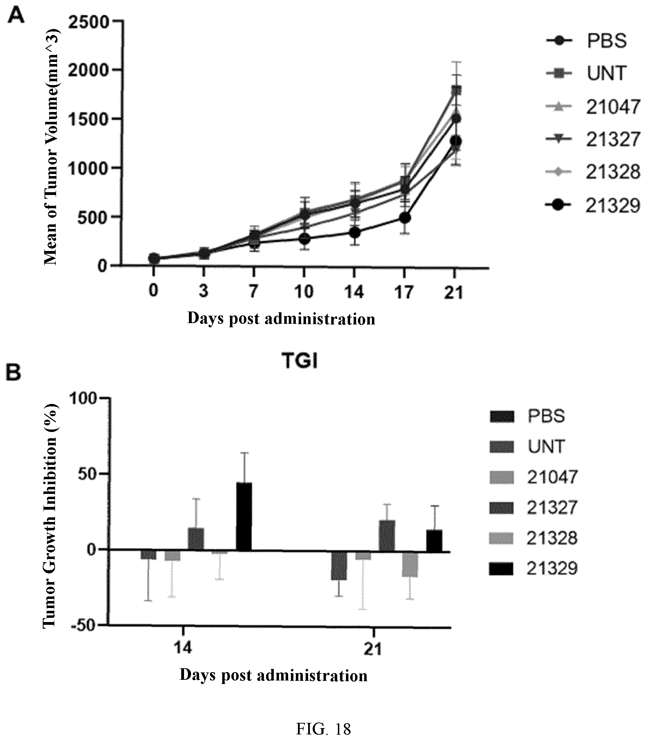

To further verify the capability of each group of SNRs to inhibit tumors, the inventors constructed a subcutaneous A431 tumor transplant model in mice (CLDN18.2-, NKG2DLs+), and CAR-T was administered to each CAR-T when a volume of the tumor reached 60 mm³, obtaining results shown in FIG. 18, in which the data showed that 21327 and 21329 could inhibit growth of A431, and 21329 had a better tumor inhibitory effect than 21327. 21047 had almost no tumor inhibitory effect, and the data also showed that the SNR structures of 21327 and 21329 could specifically recognize NKG2DL and initiate the killing effect.

### Example 12: Efficacy Experiment via CLDN18.2 Heterogeneous Tumor Model

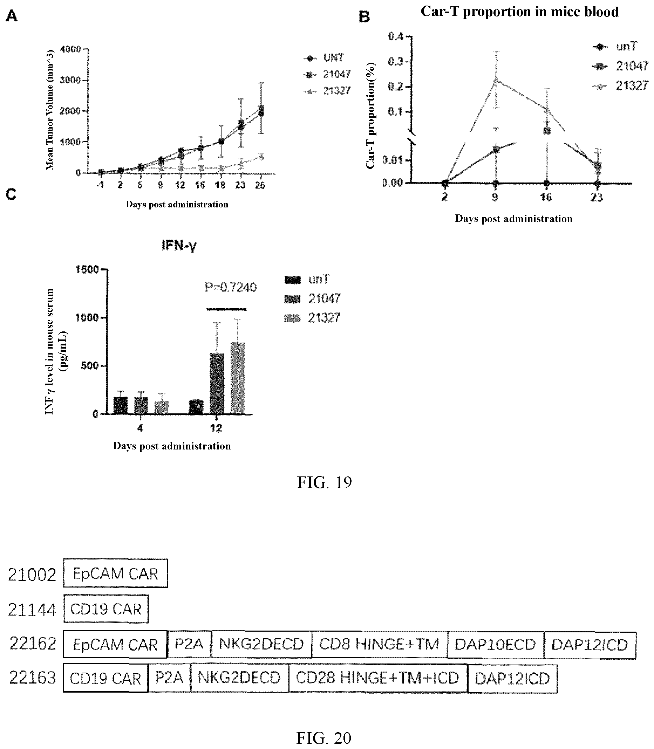

To further study whether the SNR can improve a capability of CAR-T to inhibit heterogeneous tumors, the inventors further constructed the A431-18.2 cell line and mixed the cell line with A431 to form A431/A431-18.2 tumor model. CAR-T was administered approximately 7 days after tumor inoculation. The results showed that in the heterogeneous tumor model, although conventional second-generation CAR-T molecules can proliferate and secrete IFN-gamma *in vivo,* the conventional second-generation CAR-T molecules failed to significantly inhibit tumor growth, indicating that some CLDN18.2-negative tumors could still grow normally while CLDN18.2-positive tumor cells were killed. The molecule 21327 with a SNR structure effectively inhibited growth of mixed tumor cells. Analysis results of IFN-γ secretion levels showed that there was no significant difference between secretion levels of 21047 and 21327 *in vivo* (FIG. 19). In summary, the results showed that the addition of the SNR effectively helped CAR-T cells to inhibit heterogeneous tumor tissues.

### Example 13: Construction of SNR-armed EPCAM CAR-T Cells and CD19 CAR-T Cells

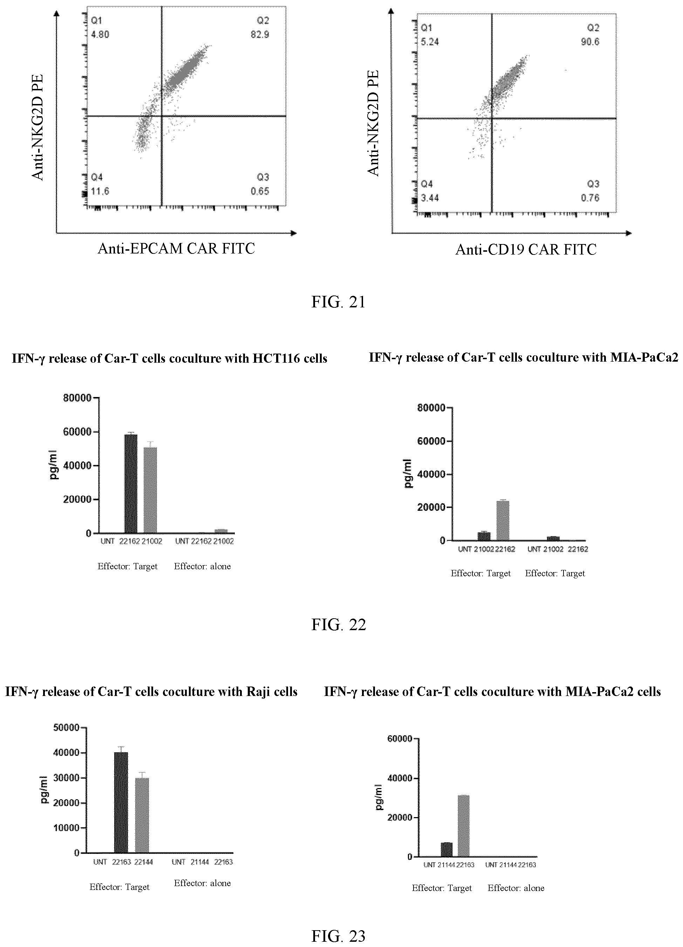

Based on the above results, the inventors constructed SNR of 21327 into a second-generation EPCAM CAR molecule (21002) to form a new CAR molecule 22162, and also constructed SNR of 21329 into a second-generation CD19 CAR molecule (21144) to form 22163 (FIG. 20). The sequence is shown in the table below. Referring to the method in Example 2, the inventors constructed plasmids, lentiviruses, and CAR-T cells with each of the above molecules. The packaged lentivirus was used to infect T cells, and the expression of anti-EPCAM-CAR, anti-CD19 CAR, and NKG2D in the T cells was detected. The results are shown in FIG. 21.

It can be seen from FIG. 21 that anti-EPCAM-CAR-T cells showed a higher NKG2D expression level, and SNR-armed anti-CD19-CAR-T cells also had a higher NKG2D expression level, while expressing EPCAM CAR and CD19 CAR normally. This indicates that the NKG2D chimeric receptor was successfully expressed on the surfaces of SNR-armed CAR-T cells.

| **Sequence/Plasmid name** | **Amino acid sequence** |
|---|---|
| 21002 | MLLLVTSLLLCELPHPAFLLIPQVQLVQSGAEVKKPG ASVKVSCKASGYTFTNYWINWVRQAPGQGLEWIGNI YPSYIYTNYNQEFKDKVTLTVDESTSTAYMELSSLRS EDTAVYYCTRSPYGYDEYGLDYWGQGTTVTVSSGG GGSGGGGSGGGGSDIQLTQSPSSLSASVGDRVTMTCK SSQSLLNTRNQKNYLTWYQQKPGKAPKLLIYWASTR ESGVPSRFSGSGSGTDFTLTISSLQPEDFATYYCQNDY VYPLTFGQGTKLEIKIEVMYPPPYLDNEKSNGTIIHVK GKHLCPSPLFPGPSKPFWVLVVVGGVLACYSLLVTVA FIIFWVRSKRSRLLHSDYMNMTPRRPGPTRKHYQPYA PPRDFAAYRSRVKFSRSADAPAYQQGQNQLYNELNL GRREEYDVLDKRRGRDPEMGGKPRRKNPQEGLYNE LQKDKMAEAYSEIGMKGERRRGKGHDGLYQGLSTAT KDTYDALHMQALPPR (SEQ ID NO. 24) |
| 22162 | MLLLVTSLLLCELPHPAFLLIPQVQLVQSGAEVKKPG ASVKVSCKASGYTFTNYWINWVRQAPGQGLEWIGNI YPSYIYTNYNQEFKDKVTLTVDESTSTAYMELSSLRS EDTAVYYCTRSPYGYDEYGLDYWGQGTTVTVSSGG GGSGGGGSGGGGSDIQLTQSPSSLSASVGDRVTMTCK SSQSLLNTRNQKNYLTWYQQKPGKAPKLLIYWASTR ESGVPSRFSGSGSGTDFTLTISSLQPEDFATYYCQNDY VYPLTFGQGTKLEIKIEVMYPPPYLDNEKSNGTIIHVK GKHLCPSPLFPGPSKPFWVLVVVGGVLACYSLLVTVA FIIFWVRSKRSRLLHSDYMNMTPRRPGPTRKHYQPYA PPRDFAAYRSRVKFSRSADAPAYQQGQNQLYNELNL GRREEYDVLDKRRGRDPEMGGKPRRKNPQEGLYNE LQKDKMAEAYSEIGMKGERRRGKGHDGLYQGLSTAT KDTYDALHMQALPPRGSGATNFSLLKQAGDVEENPG PMALPVTALLLPLALLLHAARPFNQEVQIPLTESYCGP CPKNWICYKNNCYQFFDESKNWYESQASCMSQNAS LLKVYSKEDQDLLKLVKSYHWMGLVHIPTNGSWQW EDGSILSPNLLTIIEMQKGDCALYASSFKGYIENCSTPN |
| | TYICMQRTVTTTPAPRPPTPAPTIASQPLSLRPEACRPA AGGAVHTRGLDFACDIYIWAPLAGTCGVLLLSLVITL YCLCARPRRSPAQEDGKVYINMPGRGYFLGRLVPRG RGAAEAATRKQRITETESPYQELQGQRSDVYSDLNT QRPYYK (SEQ ID NO. 25) |
| 21144 | MLLLVTSLLLCELPHPAFLLIPDIQMTQTTSSLSASLG DRVTISCRASQDISKYLNWYQQKPDGTVKLLIYHTSR LHSGVPSRFSGSGSGTDYSLTISNLEQEDIATYFCQQG NTLPYTFGGGTKLEITGSTSGSGKPGSGEGSTKGEVK LQESGPGLVAPSQSLSVTCTVSGVSLPDYGVSWIRQPP RKGLEWLGVIWGSETTYYNSALKSRLTIIKDNSKSQV FLKMNSLQTDDTAIYYCAKHYYYGGSYAMDYWGQ GTSVTVSSAAAIEVMYPPPYLDNEKSNGTIIHVKGKH LCPSPLFPGPSKPFWVLVVVGGVLACYSLLVTVAFIIF WVRSKRSRLLHSDYMNMTPRRPGPTRKHYQPYAPPR DFAAYRSRVKFSRSADAPAYQQGQNQLYNELNLGRR EEYDVLDKRRGRDPEMGGKPRRKNPQEGLYNELQK DKMAEAYSEIGMKGERRRGKGHDGLYQGLSTATKDT YDALHMQALPPR (SEQ ID NO. 26) |
| 22163 | MLLLVTSLLLCELPHPAFLLIPDIQMTQTTSSLSASLG DRVTISCRASQDISKYLNWYQQKPDGTVKLLIYHTSR LHSGVPSRFSGSGSGTDYSLTISNLEQEDIATYFCQQG NTLPYTFGGGTKLEITGSTSGSGKPGSGEGSTKGEVK LQESGPGLVAPSQSLSVTCTVSGVSLPDYGVSWIRQPP RKGLEWLGVIWGSETTYYNSALKSRLTIIKDNSKSQV FLKMNSLQTDDTAIYYCAKHYYYGGSYAMDYWGQ GTSVTVSSAAAIEVMYPPPYLDNEKSNGTIIHVKGKH LCPSPLFPGPSKPFWVLVVVGGVLACYSLLVTVAFIIF WVRSKRSRLLHSDYMNMTPRRPGPTRKHYQPYAPPR DFAAYRSRVKFSRSADAPAYQQGQNQLYNELNLGRR EEYDVLDKRRGRDPEMGGKPRRKNPQEGLYNELQK DKMAEAYSEIGMKGERRRGKGHDGLYQGLSTATKDT YDALHMQALPPRGSGATNFSLLKQAGDVEENPGPM ALPVTALLLPLALLLHAARPFNQEVQIPLTESYCGPCP KNWICYKNNCYQFFDESKNWYESQASCMSQNASLL KVYSKEDQDLLKLVKSYHWMGLVHIPTNGSWQWED GSILSPNLLTIIEMQKGDCALYASSFKGYIENCSTPNTY ICMQRTVTTTPAPRPPTPAPTIASQPLSLRPEACRPAAG GAVHTRGLDFACDIYIWAPLAGTCGVLLLSLVITLYCL |
| | CARPRRSPAQEDGKVYINMPGRGYFLGRLVPRGRGA AEAATRKQRITETESPYQELQGQRSDVYSDLNTQRPY YK (SEQ ID NO. 27) |

### Example 14: Detection of Cytokine Secretion Level of SNR-armed EpCAM CAR-T Cells

In order to verify the capability of the SNR-armed EpCAM CAR-T cells to kill heterogeneous tumors, inventors studied the cytokine release level during their killing of EpCAM target-positive cells (HCT116) or target-negative cells (MIA PaCa2).

CAR-T cells were mixed with HCT116 or MIA PaCa2 in an effect/target ratio of 1:1, the mixture was incubated for 24 hours, and then the supernatant was collected to detect a cytokine secretion level via ELISA. The IFN-γ test results are shown in FIG. 22. When HCT116 was being killed, 50000 pg/mL to 60000 pg/mL of IFN-γ was secreted by 22162, which was equivalent to the secretion level of the conventional second-generation CAR-T molecules. During the killing of EpCAM target-negative cells MIA PaCa2, SNR increased the IFN-γ secretion level of the CAR-T cells. In summary, SNR promoted EpCAM CAR-T's capability to kill EpCAM-negative cells.

### Example 15: Detection of Target Cell-Killing Capability of SNR-armed CD19 CAR-T Cells

In order to verify the capability of SNR-armed CD19 CAR-T cells to kill heterogeneous tumors, inventors studied the cytokine release level during their killing of CD19 target-positive (Raji) cells or target-negative cells (MIA PaCa2).
CAR-T cells were mixed with Raji or MIA PaCa2 in an effect/target ratio of 1:1, the mixture was incubated for 24 hours, and then the supernatant was collected to detect a cytokine secretion level via ELISA. The IFN-γ test results showed that when Raji was being killed, 30000 pg/mL to 40000 pg/mL of IFN-γ was secreted by 22163, which was significantly higher than the secretion level of the conventional second-generation CAR-T molecules. During the killing of CD19 target-negative cells MIA PaCa2, SNR increased the IFN-γ secretion level of the CAR-T cells. In summary, SNR promoted CD19 CAR-T's capability to kill CD19-negative cells.

Specific embodiments of the present disclosure are described above in detail, but are only used as examples, and the present disclosure is not limited to the specific embodiments described above. For those skilled in the art, any equivalent modification and replacement of the present disclosure also fall within the scope of the present disclosure. Therefore, equal transformations and modifications made without departing from the spirit and scope of the present disclosure shall all fall within the scope of the present disclosure.

## Claims

1. A novel chimeric receptor composition or fusion protein, comprising a chimeric antigen receptor and a NKG2D chimeric receptor comprising a full-length sequence or a truncated fragment of NKG2D, DAP10, and/or DAP12.

2. The chimeric receptor composition or fusion protein according to claim 1, comprising the chimeric antigen receptor and the NKG2D chimeric receptor, wherein the NKG2D chimeric receptor comprises sequences of a NKG2D extracellular domain and a DAP12 intracellular domain.

3. The chimeric receptor composition or fusion protein according to claim 2, wherein the NKG2D chimeric receptor further comprises a costimulatory molecule selected from a group consisting of CD28, 4-1BB, DAP10, ICOS, OX40 and CD40.

4. The chimeric receptor composition or fusion protein according to claim 1, wherein the chimeric antigen receptor comprises an extracellular recognition domain, an extracellular hinge domain, a transmembrane domain and an intracellular signal domain.

5. The chimeric receptor composition or fusion protein according to claim 4, wherein the extracellular recognition domain of the chimeric antigen receptor comprises an antibody or a fragment thereof which recognizes a tumor-associated antigen or a tumor-specific antigen preferably selected from a group consisting of CD19, BCMA, CD22, CD20, CD123, CD30, CD38, CD138, CD56, CD7, CLL-1, CD10, CD34, CS1, CD16, CD4, CD5, IL-1-RAP, ITGB7, k-IgG, TAC1, TRBC1, MUC1, NKG2D, PD-L1, CD133, CD177, LeY, CD70, ROR1, AFP, AXL, CD80, CD86, DLL3, DR5, FAP, LMP1, MAGE-A1, MAGE-A4, MG7, MUC16, PMEL, ROR2, VEGFR2, CD171, Claudin 18.2, Claudin 6, EphA2, ErbB, Fra, PSCA, cMet, IL13Ra2, EPCAM, EGFR, PSMA, EGFRvIII, GPC3, CEA, HER2, GD2, and Mesothelin.

6. The chimeric receptor composition or fusion protein according to claim 4, wherein the hinge domain has a sequence derived from at least one of CD8α, CD28, 4-1BB, ICOS, OX40, CD40, CD80, and IgG; the transmembrane domain has a sequence derived from at least one of CD2, CD27, LFA-1, CD8α, CD28, 4-1BB, ICOS, OX40, CD40, CD80, CD3ζ, and CD3ε; and the intracellular signal domain has a sequence derived from at least one of a Toll-like receptor, CD2, CD27, LFA-1, CD8α, CD28, 4-1BB, ICOS, OX40, CD40, CD80, DAP10, DAP12, CD3ζ, and CD3ε.

7. The chimeric receptor composition or fusion protein according to claim 1, further comprising a connecting peptide for connecting the chimeric antigen receptor with the chimeric receptor, wherein the connecting peptide is a self-cleaving polypeptide, i.e., a 2A peptide, including foot-and-mouth disease virus (FMDV) (F2A) peptide, porcine teschovirus (PTV-1) (P2A) peptide, Thosea asigna virus (TaV) (T2A) peptide and equine rhinitis A virus (ERAV) (E2A) peptide, preferably, the connecting peptide is P2A having an amino acid sequence of SEQ ID NO. 9.

8. The chimeric receptor composition or fusion protein according to claim 1, wherein an amino acid sequence of the chimeric receptor is one selected from SEQ ID NO. 1 to SEQ ID NO. 8, and preferably SEQ ID NO. 3 or SEQ ID NO. 6.

9. A nucleic acid encoding the chimeric receptor composition or fusion protein according to any one of claims 1 to 8.

10. A vector comprising the nucleic acid according to claim 9.

11. A cell expressing the chimeric receptor composition or fusion protein according to any one of claims 1 to 8, or comprising the nucleic acid according to claim 10 or the vector according to claim 11.

12. The cell according to claim 11, wherein the cell is one selected from a group consisting of T cells, NK cells, denritic cells (DCs), and macrophages; and preferably, the cell is an a0 T cell, a γδ T cell, or a NKT cell.

13. A biological agent for treating a tumor, comprising the cell according to claim 11 or 12 as a main active ingredient.

14. A method for preparing the cell according to claim 11 or 12, comprising a step of transfecting the cell with the vector according to claim 11.

15. Use of the chimeric receptor composition or fusion protein according to any one of claims 1 to 8, the nucleic acid according to claim 9, the vector according to claim 10, or the cell according to claim 11 or 12 for preparation of an antineoplastic drug.

16. The use according to claim 15, wherein the antineoplastic drug is used for the treatment of a heterogeneous tumor.

17. The use according to claim 16, wherein the heterogeneous tumor comprises at least NKG2DL-positive tumor cells and tumor cells targeted by the chimeric antigen receptor.
